# EUROPEAN PATENT SPECIFICATION

(11) **EP 2 061 459 B1**
(45) Date of publication and mention of the grant of the patent: **26.12.2012**
(21) Application number: 07814389.8
(22) Date of filing: 23.08.2007
(51) Int. Cl.: A61K 31/405, C07D 209/18

(54) **3-(3-INDOLYL) PROPIONIC ACID CALCIUM SALT AND METHOD OF MAKING 3-(3-INDOLYL) PROPIONIC ACID FREE ACID THEREFROM**
3-(3-INDOLYL)PROPIONSÄURE-CALCIUMSALZ UND VERFAHREN ZUR HERSTELLUNG VON 3-(3-INDOLYL)PROPIONSÄUREFREIER SÄURE DARAUS
SEL DE CALCIUM DE L'ACIDE 3-(3-INDOLYL)PROPIONIQUE ET MÉTHODE DE FABRICATION DE L'ACIDE LIBRE DE L'ACIDE 3-(3-INDOLYL)PROPIONIQUE À PARTIR DUDIT SEL

(30) Priority: 23.08.2006 US 839981 P
(43) Date of publication of application: 27.05.2009
(73) Proprietor: Intellect Neurosciences Inc., New York, NY 10011 (US)
(72) Inventor: ROGERS, Norman, H., Horsham West Sussex RH13 5NY (GB)
(74) Representative: Grünecker, Kinkeldey, Stockmair & Schwanhäusser
(86) International application number: PCT/US2007/076645
(87) International publication number: WO 2008/024914

(56) References cited:
- WO-A1-99/42102
- WO-A2-2006/018850
- CN-A- 1 273 776

## Description

Alzheimer's Disease ("AD") is the most common cause of dementia in the elderly. It has been estimated that AD affects up to 7% of people over the age of 65 and 40% of people over the age of 80. Because the elderly are the fastest growing segment of society, the number of people with AD in the United States and the cost incurred to care for them is predicted to triple within the next 25 years. Thus, AD is a major public health problem that will grow in the foreseeable future.

AD is characterized by the appearance of structural abnormalities in the brain. These include cytoskeletal abnormalities in neurons and the appearance of amyloid deposits in senile plaques. "Amyloid" is the histological term for AD-related fibrillar peptides arranged in aggregated β-sheets that are doubly refractive when stained and viewed in polarized light. The principal components of amyloid are peptides that are 40 to 42 amino acids in length called the amyloid beta ("Aβ") peptides (Aβ 1-40, Aβ 1-41, and Aβ 1-42, respectively). The Aβ peptides are derived from a larger precursor known as amyloid precursor protein ("APP"), and are also produced during normal cellular metabolism. The Aβ peptides have a common N-terminus but differ at their respective C-termini.

There is currently no cure for AD. AD therapies have been focused on alleviating symptoms associated with the disease, such as depression, agitation, sleep disorders, hallucinations and delusions. The basal forebrain cholinergic system, a region of the brain that is severely damaged in AD, has been a principal target of some experimental therapies. Attempts to influence the cholinergic system, however, have been generally ineffective.

Other attempts to treat AD focus on preventing or ameliorating Aβ protein deposition. Aβ protein is toxic to neurons, providing a possible connection between amyloid accumulation and neurodegeneration. Because of the close association between AD and aging and the similarities in the neuropathologies of both conditions, oxidative stress has been proposed to play a role in the development of the lesions characteristic of AD. Oxygen free radicals may be related to the cytotoxicity of Aβ protein. Indeed, markers of oxidative injury are topographically associated with the neuropathologic lesions present in AD patients. As a result, antioxidants have been proposed as possible therapeutic agents.

3-(3-indolyl)propionic acid ("3-IPA," which is also known as indole-3-propionic acid or OXIGON™) is a naturally-occurring, potent antioxidant that acts as a radical scavenger without generating pro-oxidative intermediates. 3-IPA has been shown to protect cultured neurons from the toxicity of the Aβ peptide and is currently being developed as a disease-modifying therapy for AD. U.S. Patent No. 6,395,768 discloses the use of 3-IPA in the free acid, salts or ester forms, for inhibiting the cytotoxic effects of Aβ protein on cells, for treating fibrillogenic diseases and for protecting cells from oxidative damage. Disclosed suitable salts include pharmaceutically acceptable salts such as sodium salts, potassium salts and ammonium salts.

3-IPA is manufactured world-wide on an industrial scale. One method for synthesizing 3-IPA is by the deamination of tryptophan. This method, however, is not commercially viable because of the high cost of the tryptophan starting material and the production of a potentially explosive diazo intermediate, requiring the use of special facilities and equipment for synthesis on an industrial scale. Another method used to synthesize 3-IPA involves the initial preparation of a Meldrum's adduct intermediate in the presence of proline. See Rajeswaren J. Org. Chem. 199, 64, 1369. However proline is considered to be too expensive to be used in the amounts consumed in the production-scale reaction.

The standard, currently used method for large scale production of 3-IPA is a one-step, one-pot process that utilizes indole, acrylic acid, acetic anhydride and acetic acid as starting materials. This method has several drawbacks. The method typically requires considerable process optimization to produce reasonable yields of 3-IPA. Moreover, the IPA produced is relatively impure and, thus, is unsuitable for use as a pharmaceutical ingredient until it is purified. Purification of the 3-IPA to a purity level that is acceptable for an active pharmaceutical ingredient is a time-consuming process requiring a number of recrystallization steps and the use (and subsequent disposal) of large volumes of solvents that are used for crystallization.

Thus, there is a need for new and improved commercially viable means of synthesizing 3-IPA in large yields and with an acceptable purity, for use as an active pharmaceutical ingredient in pharmaceutical formulations.

It has now been surprisingly discovered that the calcium salt of 3-IPA is much less soluble in water compared to the calcium salts of other acids present in a reaction mixture for the synthesis of 3-IPA (*e.g*., acrylic acid or acetic acid). The low solubility in water of 3-IPA calcium salt is surprising because many other salts of 3-IPA, such as the sodium salt, are highly soluble in water. The low comparative solubility of the calcium salt of 3-IPA allows for easy removal of more soluble contaminants from the solid salt, which may then be converted subsequently to substantially pure 3-IPA. The methods of making 3-IPA with 3-IPA calcium as an intermediate improve upon existing methods of making pure 3-(3-indolyl)propionic acid. The prior methods of making 3-IPA require the use of large volumes of solvent for repeated extractions to remove acrylic acid and other contaminants from the product, and to avoid time-consuming and costly purification by filtration through silica gel, which would be problematic to conduct on a large scale.
The present invention relates to a compound 3-(3-indolyl)propionic acid calcium salt or a hydrate thereof.

In certain embodiments the invention provides the calcium salt of 3-IPA ("3-IPA calcium"). In certain preferred embodiments the invention provides 3-IPA calcium in a substantially pure form.

In certain embodiments the invention provides methods of making substantially pure 3-IPA free acid from 3-IPA calcium, comprising converting 3-IPA free acid into 3-IPA calcium, precipitating and washing the 3-IPA calcium, and reconverting the 3-IPA calcium to substantially pure 3-IPA free acid. Preferred embodiments are set forth in the subclaims.

Also disclosed are compositions comprising 3-IPA calcium, preferably compositions comprising substantially pure 3-IPA calcium, more preferably compositions consisting essentially of pure 3-IPA calcium. The invention also discloses pharmaceutical compositions.

Also disclosed are methods of using 3-IPA calcium and compositions thereof to treat fibrillogenic diseases and other diseases or conditions in which free radicals or oxidative stress play a role. 3-IPA calcium and compositions thereof may be used to treat the cytotoxic effects of amyloid beta protein on cells.

Also disclosed are methods of using 3-IPA calcium and compositions thereof for the preparation of a medicament to treat fibrillogenic diseases and other diseases or conditions in which free radicals or oxidative stress play a role. 3-IPA calcium and compositions thereof may be used for the preparation of a medicament to treat the cytotoxic effects of amyloid beta protein on cells.

The present invention relates 3-IPA calcium, which has not been previously described. Also disclosed are pharmaceutical compositions containing 3-IPA calcium and methods of treating AD and other conditions in which free radicals or oxidative stress plays a role by administering to a subject a therapeutically effective amount of 3-IPA calcium. The higher melting point of 3-IPA calcium compared to the free acid form offers significant advantages for the preparation of solid compositions, such as tablets. Without being bound by any particular theory, the relatively lower melting point of the free acid form of 3-IPA may result in fusion of the compound during tablet compression, which will not occur during compression of the calcium salt form of the compound. The low solubility of 3-IPA calcium compared to the free acid form offers significant advantages for the preparation of formulations for sustained release, delivery by depot and injection.

In addition, the present invention relates to a method of preparing the free acid of 3-IPA using 3-IPA calcium. The present invention is based in part on the unexpected and surprisingly low water solubility of 3-IPA calcium.

### General Definitions

"3-IPA" is 3-(3-indolyl)propionic acid.

"3-IPA calcium" refers to the calcium salt of 3-(3-indolyl)propionic acid.

The terms "about" or "approximately" mean within an acceptable range for the particular parameter specified as determined by one of ordinary skill in the art, which will depend in part on how the value is measured or determined, e.g., the limitations of the measurement system. For example, "about" can mean a range of up to 20% of a given value. Alternatively, particularly with respect to biological systems or processes, the term can mean within an order of magnitude, preferably within 5-fold, and more preferably within 2-fold, of a value.

As used herein, the terms "treat," "treating," or "treatment" mean the reduction, amelioration, or alleviation, of AD and other fibrillogenic diseases such as, without limitation, prion-related diseases, diseases or conditions in which free radicals or oxidative stress plays a role, such as, aging, Parkinson's Disease, Huntington's Disease, Down's Syndrome, Lewy body dementia, amyotrophic lateral sclerosis, progressive supranuclear palsy, other forms of amyloidoses, stroke, atherosclerosis, emphysema, and some forms of cancer.

The term "fibrillogenic disease" as used herein includes any disease or condition involving undesirable deposition of fibrils. Such diseases or conditions include, but are not limited to, AD, other amyloidoses, and prion diseases such as Creutzfeldt-Jakob disease in humans, bovine spongiform encephalopathy in cattle and scrapie in sheep.

The terms "amyloid beta protein" or "Aβ protein" refer to the related set of peptides that are derived proteolytically from the amyloid precursor protein (APP). Examples of species included in the terms "amyloid beta protein" and "Aβ protein" are Aβ 1-40, Aβ 1-41 and Aβ 1-42, which begin at a common N-terminal residue and extend, respectively, for 40, 41 and 42 amino acids. Other proteolytically derived fragments of APP also appear in amyloid plaques and these peptides are also included in the terms "amyloid beta protein" and "Aβ protein."

The term "subject" refers to a mammal (*e.g*., any veterinary medicine patient such as a domesticated animal, such as a dog or cat), or a human patient.

The phrase "pharmaceutically acceptable," as used in connection with compositions of the invention, refers to molecular entities and other ingredients of compositions that are physiologically tolerable and do not typically produce untoward reactions when administered to a mammal (e.g., a human). Preferably, as used herein, the term "pharmaceutically acceptable" means approved by a regulatory agency of the Federal or a state government or listed in the U.S. Pharmacopoeia or other generally recognized pharmacopeias for use in mammals, and more particularly in humans.

3-IPA calcium may be administered with one or more carriers. The term "carrier" applied to pharmaceutical compositions of the invention refers to a diluent, excipient, or vehicle with which 3-IPA calcium is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington The Science and Practice of Pharmacy," 20th edition, (Alfonso R. Gennaro, ed. 2000).

A "pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used in the present application includes both one and more than one such excipient.

A "therapeutically effective amount" means an amount of 3-IPA calcium that, when administered to a mammal for treating a state, disorder or condition, is sufficient to effect such treatment. The "therapeutically effective amount" will vary depending on the disease and its severity and the age, weight, physical condition and responsiveness of the mammal to be treated.

"Percent" or "%" as used herein refers to the percentage by weight of the total composition.

The term "substantially pure 3-(3-indolyl)propionic acid" or "substantially pure 3-IPA" refers to 3-IPA free acid of about 97.0% purity or greater, preferably about 98% purity or greater, and most preferably about 99% purity or greater.

### 3-IPA Calcium Salt

3-IPA calcium is a dibasic salt having the chemical formula:

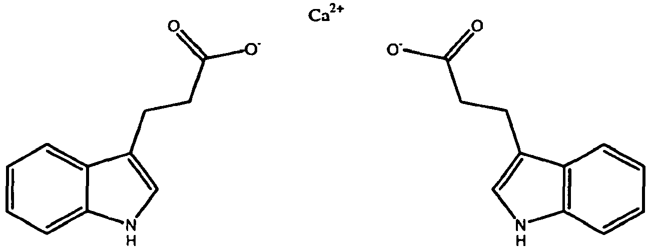

In one embodiment, 3-IPA calcium exists in the absence of water of hydration. In another embodiment, 3-IPA calcium exists as a hydrate that is associated with between 0.25 and 10 water molecules on average, per molecule of 3-IPA calcium, e.g., 3-IPA calcium sesquihydrate, dihydrate, trihydrate, etc. Thus, 3-IPA calcium has the molecular formula C₂₂H₂₀N₂O₄Ca·XH₂O, wherein X has a value between 0 and 10.

3-IPA calcium may be in a crystalline or amorphous form. In addition, crystalline forms of 3-IPA calcium may exist as polymorphs.

The present invention encompasses all polymorphs and hydrates of 3-IPA calcium.

Also disclosed is a method for treating or inhibiting the cytotoxic effects of Aβ protein on the cells of a mammal, comprising exposing the cells of said mammal to a therapeutically effective amount of 3-IPA calcium. Preferably, the cells are neuronal cells. More preferably, the neuronal cells are brain cells. Cytotoxic effects include decreased cell viability, increased lipid peroxidation, increased intracellular calcium levels, diffuse membrane blebbing, cell retraction, abnormal distribution of chromatin toward the nuclear membrane and karyorrhexis.

Also disclosed is a method of treating fibrillogenic diseases in a subject, comprising administering to the subject a therapeutically effective amount of 3-IPA calcium to inhibit the formation of fibrils. Such fibrillogenic diseases include AD, other amyloidosis diseases, and prior-related diseases.

Also disclosed is a method of treating a disease or condition in which free radicals and/or oxidative stress play a role. Such diseases or conditions include aging, Parkinson's Disease, Huntington's Disease, Down's Syndrome, Lewy body dementia, amyotrophic lateral sclerosis, progressive supranuclear palsy, other forms of amyloidoses, stroke, atherosclerosis, emphysema, and some forms of cancer.

### Compounds and Pharmaceutical Compositions

For use in therapy, 3-IPA calcium may be administered as a compound or in a pharmaceutical composition. 3-IPA calcium may be administered as a substantially pure compound. 3-IPA calcium may be administered as a compound that is 90% pure, 95% pure, 99% pure, 99.5% pure or 99.9% pure. Compositions may comprise an active ingredient consisting essentially of 3-IPA calcium. Preferably, the active ingredient consists essentially of 3-IPA calcium having a purity of 90%, 95%, 99%, 99.5% or 99.9%. Compositions may comprise 3-IPA calcium in combination with one or more additional therapeutic agents for treating, *e.g.*, AD. These additional therapeutic agents include for example, antibodies specific for one or more Aβ protein sequences.

Also disclosed is a pharmaceutical composition or formulation comprising 3-IPA calcium in association with a pharmaceutically acceptable excipient, diluent and/or carrier. The excipient, diluent and/or carrier must be "acceptable" in the sense of being compatible with the other ingredients of the formulation and not deleterious to the recipient thereof.

Also disclosed is a pharmaceutical composition comprising 3-IPA calcium in association with a pharmaceutically acceptable excipient, diluent and/or carrier for use in therapy, and in particular, in the treatment of human or animal subjects suffering from a condition susceptible to amelioration by an anti-oxidant compound.

Also disclosed is a pharmaceutical composition comprising a therapeutically effective amount of 3-IPA calcium and a pharmaceutically acceptable excipient, diluent and/or carrier (including combinations thereof).

3-IPA calcium may be formulated for administration in any convenient way for use in human or veterinary medicine. Also disclosed are pharmaceutical compositions comprising 3-IPA calcium adapted for use in human or veterinary medicine. Such compositions may be presented for use in a conventional manner with the aid of one or more suitable excipients, diluents and/or carriers. Acceptable excipients, diluents and carriers for therapeutic use are well known in the pharmaceutical art, and are described, for example, in "Remington The Science and Practice of Pharmacy," 20th edition, (Alfonso R. Gennaro, ed. 2000). The choice of pharmaceutical excipient, diluent and/or carrier can be selected with regard to the intended route of administration and standard pharmaceutical practice. The pharmaceutical compositions may comprise as - or in addition to - the excipient, diluent and/or carrier any suitable binder(s), lubricant(s), suspending agent(s), coating agent(s), solubilizing agent(s).

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

3-IPA calcium may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e. g. as a carrier, diluent or solubilizer. Alpha-, beta- and gamma-cyclodextrins are most commonly used and suitable examples are described in WO 91/11172, WO 94/02518 and WO 98/55148.

3-IPA calcium may be milled using known milling procedures such as wet milling to obtain a particle size appropriate for tablet formation and for other formulation types. Finely divided (nanoparticulate) preparations of 3-IPA calcium may be prepared by processes known in the art, for example see International Patent Publication WO 02/000196.

The routes for administration (delivery) include one or more of: oral (*e.g*., as a tablet, capsule, or as an ingestable solution), topical, mucosal (*e.g*., as a nasal spray or aerosol for inhalation), nasal, parenteral (*e.g*., by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural and sublingual. Preferable routes for administration include intramuscular, via depot formation, and rectal (by suppository).

There may be different composition/formulation requirements depending on the different delivery systems. The pharmaceutical compositions disclosed herein may be formulated to be delivered using a mini-pump or by the mucosal route, for example, as a nasal spray or aerosol for inhalation or ingestable solution, or parenterally in which the composition is formulated by an injectable form, for delivery, by, for example, an intravenous, intramuscular or subcutaneous route. The formulation may also be designed as a slow-release oral formulation.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a cosmetic, a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavoring or coloring agents, or they can be injected parenterally, for example intravenously, intramuscularly or subcutaneously. For parenteral administration, the compositions may be best used in the form of a sterile aqueous suspension, which may contain other substances, for example enough salts, such as sodium chloride, potassium chloride, or magnesium sulfate, or monosaccharides, such as glucose, galactose or fructose, to make the solution isotonic with blood. For buccal or sublingual administration the compositions may be administered in the form of tablets or lozenges, which can be formulated in a conventional manner.
The compositions disclosed herein include those in a form especially formulated for particular routes of administration, and preferably for parenteral, oral, or rectal administration. For some applications, the agents are delivered systemically (such as orally). In a particularly preferred embodiment, pharmaceutical compositions containing 3-IPA calcium as the active pharmaceutical ingredient are delivered orally. Hence, preferably the agent is in a form that is suitable for oral delivery.

If 3-IPA calcium is administered parenterally, then examples of such administration include one or more of: intravenously, intraarterially, intraperitoneally, intrathecally, intraventricularly, intraurethrally, intrasternally, intracranially, intramuscularly or subcutaneously administering the agent; and/or by using infusion techniques.

For parenteral administration, 3-IPA calcium may be used in the form of a sterile aqueous suspension which may contain other substances, for example, enough salts (such as such as sodium chloride) or glucose to make the solution isotonic with blood. The aqueous suspensions should be suitably buffered (preferably to a pH of from 7 to 9), if necessary. The preparation of suitable parenteral formulations under sterile conditions is readily accomplished by standard pharmaceutical techniques well-known to those skilled in the art.

In addition to the formulations described previously, the 3-IPA calcium may also be formulated for parenteral administration as a depot preparation. Such long acting formulations may be administered by implantation (for example subcutaneously or intramuscularly) or by intramuscular injection. Thus, for example, 3-IPA calcium may be formulated with suitable polymeric or hydrophobic materials (for example as an emulsion in an acceptable oil with appropriate surfactants) or ion exchange resins.

3-IPA calcium may be formulated for use in human or veterinary medicine by injection (e.g. by intravenous bolus injection or infusion or via intramuscular, subcutaneous or intrathecal routes) and may be presented in unit dose form, in ampoules, or other unit-dose containers, or in multi-dose containers, if necessary with an added preservative. The compositions for injection may be in the form of suspensions, solutions, or emulsions, in oily or aqueous vehicles, and may contain formulatory agents such as suspending, stabilizing, solubilizing and/or dispersing agents. Alternatively the active ingredient may be in sterile powder form for reconstitution with a suitable vehicle, e.g. sterile, pyrogen-free water, before use.

3-IPA calcium can be administered (*e.g*., orally or topically) in the form of tablets, capsules, ovules, elixirs, solutions or suspensions, which may contain flavoring or coloring agents, for immediate-, delayed-, modified-, sustained-, pulsed-or controlled-release applications. The lower solubility of 3-IPA calcium compared to the free acid form of 3-IPA makes it especially suitable for use in sustained-release oral formulations, which will have slower gut transit times.

3-IPA calcium may also be presented for human or veterinary use in a form suitable for oral or buccal administration, for example in the form of solutions, gels, syrups, mouth washes or suspensions, or a dry powder for constitution with water or other suitable vehicle before use, optionally with flavoring and coloring agents. Solid compositions such as tablets, capsules, lozenges, pastilles, pills, boluses, powder, pastes, granules, bullets or premix preparations may also be used. Solid and liquid compositions for oral use may be prepared according to methods well known in the art. Such compositions may also contain one or more pharmaceutically acceptable carriers and excipients which may be in solid or liquid form.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia.

Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, 3-IPA calcium may be combined with various sweetening or flavoring agents, coloring matter or dyes, with emulsifying and/or suspending agents and with diluents such as water, ethanol, propylene glycol and glycerin, and combinations thereof.

3-IPA calcium may also be administered orally in veterinary medicine in the form of a liquid drench such as a solution, suspension or dispersion of the active ingredient together with a pharmaceutically acceptable carrier or excipient.

The compound may also, for example, be formulated as suppositories e.g. containing conventional suppository bases (such as polyethylene glycol polymers, polyoxyl 40 stearate or polyoxyethylene sorbitan fatty acid esters) for use in human or veterinary medicine or as pessaries *e.g*., containing conventional pessary bases (such as cocoa butter, glycerol/gelatin glyco-gelatin or polyethylene glycol).

3-IPA calcium may be formulated for topical administration, for use in human and veterinary medicine, in the form of ointments, creams, gels, hydrogels, lotions, solutions, shampoos, powders (including spray or dusting powders), pessaries, tampons, sprays, dips, aerosols, drops (*e.g*., eye ear or nose drops) or pour-ons.

For application topically to the skin, 3-IPA calcium can be formulated as a suitable ointment in which the compound is dissolved in, for example, a mixture with one or more of the following: mineral oil, liquid petrolatum, white petrolatum, propylene glycol, polyoxyethylene polyoxypropylene compound, emulsifying wax and water.

3-IPA calcium can also be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

3-IPA calcium may also be dermally or transdermally administered, for example, by use of a skin patch.

As indicated, 3-IPA calcium can be administered intranasally or by inhalation and is conveniently delivered in the form of a dry powder inhaler or an aerosol spray presentation from a pressurized container, pump, spray or nebulizer with the use of a suitable propellant, e.g. dichlorodifluoromethane, trichlorofluoromethane, dichlorotetrafluoroethane, a hydrofluoroalkane such as 1,1,1,2-tetrafluoroethane (HFA 134AT"") or 1,1,1,2,3,3,3-heptafluoropropane (HFA 227EA), carbon dioxide or other suitable gas. In the case of a pressurized aerosol, the dosage unit may be determined by providing a valve to deliver a metered amount. The pressurized container, pump, spray or nebuliser may contain a solution or suspension of 3-IPA calcium, *e.g*., using a mixture of ethanol and the propellant as the solvent, which may additionally contain a lubricant, e. g. sorbitan trioleate.

Capsules and cartridges (made, for example, from gelatin) for use in an inhaler or insufflator may be formulated to contain a powder mix of 3-IPA calcium and a suitable powder base such as lactose or starch.

### Dosages

Typically, a physician will determine the actual dosage which will be most suitable for an individual subject. The specific dose level and frequency of dosage for any particular individual may be varied and will depend upon a variety of factors including the age, body weight, general health, sex, diet, mode and time of administration, rate of excretion, drug combination, the severity of the particular condition, and the individual undergoing therapy.

For oral and parenteral administration to humans, the daily dosage level of the agent may be in single or divided doses. The amount of 3-IPA calcium effective for treating various diseases or conditions can be determined by conventional methods.

### Indications

3-IPA calcium may be used to treat any condition that is caused, at least in part, by oxidative stress. 3-IPA calcium containing dosage forms are used to treat fibrillogenic diseases. Examples of fibrillogenic diseases include any disease or condition involving undesirable deposition of fibrils, such as amyloidosis diseases and prion-related diseases such as Creutzfeldt-Jakob disease and Gerstmann-Straussler-Scheinker disease in humans, bovine spongiform encephalopathy in cattle and scrapie in sheep.

3-IPA calcium containing dosage forms are useful in treating diseases where free radicals and/or oxidative stress play a role, such as, aging, Parkinson's Disease, Huntington's Disease, Down's Syndrome, Lewy body dementia, amyotrophic lateral sclerosis, progressive supranuclear palsy, amyloidosis diseases, atherosclerosis, emphysema, some forms of cancer, emphysema, asthma, diabetes and its consequences (*e.g.* retinopathy and nephropathy), exercise-induced tissue damage and physical performance, autoimmune diseases, epilepsy, polyneuropathies, hepatic disorders, AIDS, macular degeneration, myelodysplastic syndrome, damage caused by chemotherapy, ataxia-telangiectasia and diseases caused by defective DNA repair genes. 3-IPA calcium containing formulations are also useful in providing protection against damage caused by ionizing radiation. 3-IPA calcium containing dosage forms may also be used to treat acute conditions such as trauma from injuries such as head injuries, and ischemic and reperfusion injuries such as stroke.

Controlled-release 3-IPA calcium containing dosage forms may be used to treat AD.

### Synthesis of 3-IPA Free Acid from 3-IPA calcium

3-IPA free acid can be synthesized on a large scale in a one-step, one-pot process from the starting materials indole, acrylic acid, acetic anhydride and acetic acid. *See* H E Johnson and D G Crosby (1960) J. Org. Chem. 25:569. This process produces poor yields and much effort must be put into optimizing it before reasonable yields are achieved. The purity of 3-IPA produced by this process is unacceptable if the compound is to be used as an active pharmaceutical ingredient. Thus, further purification (e.g., by crystallization) is required. Purification requires the manipulation and subsequent disposal of large volumes of solvents for recrystallization which are toxic to individuals and detrimental to the environment.

New methods of making 3-IPA free acid from 3-IPA calcium as described herein result in a higher purity of the free acid. The new method comprises the steps of converting 3-IPA free acid to the calcium salt, which has a surprisingly low solubility in water compared to the solubility of the calcium salts of the acrylic acid and acetic acid starting materials. Thus, 3-IPA calcium precipitates from the aqueous solution, is collected by filtration and washed several times with water to remove water-soluble impurities, *i.e*., acrylic acid calcium salt and acetic acid calcium salt. The pure 3-IPA calcium is then reconverted to the free acid.

3-IPA free acid can be converted to the calcium salt by any method known in the art. For example, calcium chloride can be added to a solution of 3-IPA free acid to make the calcium salt, which is less soluble than the calcium salts of other acids present in the solution and will precipitate out of the solution. Other suitable calcium salts include calcium nitrate, calcium acetate, calcium hydroxide, calcium carbonate, calcium phosphate, and hydrates thereof. Preferably, calcium acetate or calcium chloride is used in the methods of the invention.

3-IPA free acid can be converted to the calcium salt at a pH of 6-7 or higher in the presence of ammonium hydroxide by any method known in the art. For example, calcium chloride can be added to a solution of 3-IPA free acid in aqueous ammonium hydroxide to form the calcium salt, which due to its lower solubility compared to calcium salts of other acids present in the solution, will precipitate from the solution. Other suitable bases for use in the methods of forming the calcium salt of 3-IPA include tetramethylammonium hydroxide, tetraethylammonium hydroxide, EDTA, tetrabutylammonium hydroxide, and tetrapropylammonium hydroxide.

3-IPA calcium can be isolated by any method known in the art. In general 3-IPA calcium is isolated by filtration, washed first with water and then with a solvent such as ethanol, isopropanol, ethyl acetate or T-butyl methyl ester to remove water and soluble impurities such as calcium acrylate and other neutral impurities present, and dried by, *e.g.,* a vacuum or air drying.

3-IPA calcium can be converted to the free acid by any method known in the art. In general, the calcium salt can be converted to the free acid by adding an aqueous solution of an excess of acid such as acetic acid to the calcium salt. Other suitable acids for use in the method of the invention include hydrochloric acid, hydrobromic acid, hydroiodic acid, perchloric acid, nitric acid, sulfonic acid and sulfuric acid. Preferably, acetic acid is used in the method of the invention to convert 3-IPA acid calcium salt into the substantially pure free acid.

The substantially pure 3-IPA free acid can be isolated by extraction with a suitable polar organic solvent such as ethyl acetate or methylene chloride, or preferably, by precipitation. Precipitation may be induced by, *e.g*., diluting the solution of 3-IPA free acid with water and subsequently adding a water soluble organic solvent, such as ethanol or methanol. Any solvent or solvent system suitable for recrystallization of 3-IPA free acid may be used in this step. After precipitation and separation of the solid from the mother liquor, *e.g*., by vacuum filtration, 3-IPA free acid is washed and then dried.

### EXAMPLES

### Example 1

### Synthesis of 3-IPA using acrylic acid and a 3-IPA calcium Intermediate for Purification

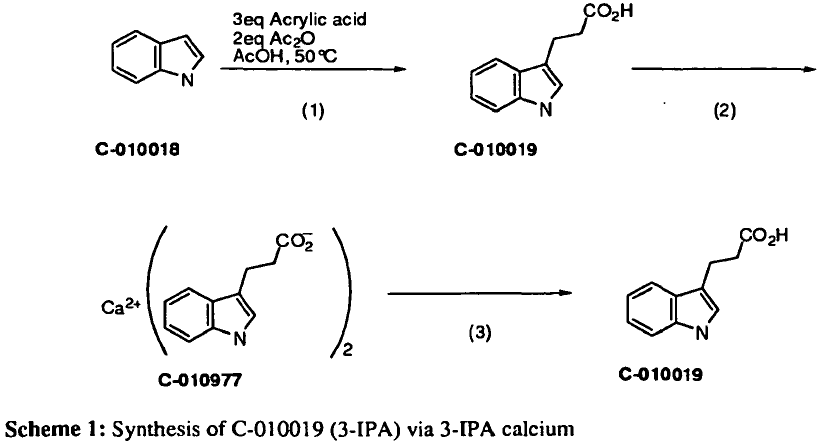

Indole (8.015 kg), acetic acid (16 L), acrylic acid (3 eq., 14L) and acetic acid anhydride (2 eq., 13 L) were heated for 64 hours to 50°C under a nitrogen atmosphere. (Step 1, Scheme 1). The indole showed a conversion to 3-IPA of 98.6%. The reaction mixture was cooled to 18-20°C, water (22.5 L) was added and stirring was continued for 18 hours at 18-20°C. The resulting mixture showed no residual anhydrides, and the purity of 3-IPA was found to be 69.32 area % by high performance liquid chromatography (HPLC). Heptane (17.2 L) was added and 45 L of acetic acid / water were removed by azeotropic distillation (p= 90-140 mbar, IT= 28-32°C, OT=60-70°C). 17 L of heptane were removed by distillation (p=54-80 mbar, OT=60°C, IT=33-42°C). When the temperature of the reaction mixture reached 20°C, ethyl acetate (25 L) and water (69 L) were added. At a temperature between 0-10°C, the pH was adjusted to 12 using 30% sodium hydroxide (32 L). After phase separation at a temperature of 4-7°C, the aqueous layer was extracted with ethyl acetate (2x25 L). Ethyl acetate (32 L) was added to the aqueous phase and the pH was adjusted to 6-7 using 32% HCl (0.8 L) at 4-7°C.

When the water/ethyl acetate mixture reached a temperature of 20°C, a saturated calcium acetate solution (39 L) was added within 1 hour and a suspension was formed. (Step 2, Scheme 1). The suspension was stirred at 15°C for 15 hours. 3-IPA calcium was collected by filtration and washed with a saturated calcium acetate solution (12 L), water (40 L) and ethyl acetate (40 L). The wet filter cake (containing 99.5 area % 3-IPA calcium by HPLC) was dissolved in acetic acid (31 L) at 18-20°C and water (88 L) was added within 1 hour. (Step 3, Scheme 1). The suspension was stirred overnight at 10°C. Crude 3-IPA was collected by filtration and washed with water (24 L). The wet filter cake was dissolved in isopropyl alcohol (44 L) and water (120 L) was added within 1 hour at 20-25°C. The suspension was cooled to 5°C and stirred for 65 hours. 3-IPA was isolated by filtration, washed with water (32 L) and dried. 4.7 kg (36% yield, 99.87 area%, 98.89 w/w %).

### Example 2

### Synthesis of 3-IPA using acrylic acid and a 3-IPA calcium Intermediate for Purification

Indole (58.59 g), acetic acid (120 mL), acrylic acid (3 eq., 103 mL) and acetic acid anhydride (2eq., 94 mL) were heated for 43 hours to 50°C under a nitrogen atmosphere. (Step 1, Scheme 1). The indole showed a conversion to 3-IPA of 97.9%. The reaction mixture was cooled to 20°C, water (164 mL) was added, and stirring was continued for 18 hours at 20°C. The resulting mixture showed no residual anhydrides. The purity of 3-IP A was found to be 72.12 area % by HPLC. Heptane (126 mL) was added and 230 mL of the acetic acid/water mixture was removed by azeotropic distillation (p=119 mbar, IT=21-40°C, OT=70°C). 92 mL of heptane were removed by distillation (119 mbar, OT=70°C, IT=40-50°C). When the temperature of the reaction mixture reached 20°C, ethyl acetate (182 mL) and water (502 mL) were added. When the reaction mixture reached a temperature between 5°C and 11°C, 30% sodium hydroxide (109 mL) was added and the pH was adjusted to 8.5 using 1M hydrochloric acid (3.5 mL). After phase separation occurred at a temperature of 15°C, the aqueous layer was extracted with ethyl acetate (2x180mL). The pH was adjusted to 7.7 using acetic acid/water (1:1), and ethyl acetate (235 mL) was added at 15°C.

When the water/ethyl acetate mixture reached a temperature of 20°C, a saturated calcium chloride solution (224 mL) was added within 1 hour and a suspension formed. (Step 2, Scheme 1). The suspension was stirred at 15°C for 15 hours. 3-IPA calcium was collected by filtration and washed with a saturated calcium acetate solution (70 mL), water (295 mL) and ethyl acetate (295 mL). 29.3 g of the resulting wet filter cake (containing 98.7 area % of 3-IPA calcium by HPLC) was dissolved in acetic acid (115 mL) at 20°C and water (325 mL) was added within 5 minutes. (Step 3, Scheme 1). The suspension was heated to 71°C and a clear solution was obtained. The reaction mixture was cooled down to 0°C within 4 hours. The suspension was stirred overnight at 0°C. Crude 3-IPA was collected by filtration and was washed with water (88 mL). The wet filter cake was dissolved in isopropyl alcohol (160 mL) and water (440 mL) was added within 1 hour at 20-25°C. The suspension was cooled to 5°C and stirred overnight. 3-IPA was isolated by filtration, washed with water (116 mL) and dried. 16.6 g (35% yield, 99.7 area% by HPLC).

### Example 3

### Synthesis of 3-IPA using a 3-IPA calcium Intermediate for Purification

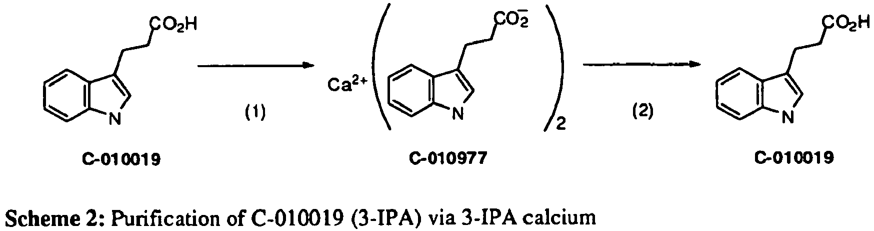

14.421 kg 3-IPA (97.4 area % by HPLC) were dissolved in water (51 L) and ammonium hydroxide solution (12.6 L, 25%). The resulting solution had a pH of 9. Ethyl acetate (51 L) was added. When the water/ethyl acetate mixture reached a temperature of 20°C, a saturated calcium chloride solution (20 L) was added within 1 hour and a suspension was formed. (Step 1, Scheme 3). The suspension was stirred at 20°C for 14 hours. 3-IPA calcium was collected by filtration and washed with water (14.5 L) and ethyl acetate (2x14.5 L). The wet filter cake containing 99.74 area % 3-IPA calcium as determined by HPLC was dissolved in acetic acid (50.5 L) at 18-19°C and water (143 L) was added within 1 hour. (Step **2**, Scheme 2). The suspension was heated to 75°C and a clear solution was obtained. The reaction mixture was cooled down to 0°C within 4 hours. The suspension was stirred overnight at 0°C. Crude 3-IPA was collected by filtration and washed with water (39 L). The wet filter cake was dissolved in isopropyl alcohol (126 L) and water (366 L) was added within 1 hour at 20-22°C. The suspension was cooled to 0°C and stirred overnight. 3-IPA was isolated by filtration, washed with water (117 L) and dried 11.677 kg (81% recovery, 99.8 area % by HPLC).

## Claims

1. 3-(3-Indolyl)propionic acid calcium salt or a hydrate thereof.

2. A method of making substantially pure 3-(3-indolyl)propionic acid free acid, comprising the steps of:
(a) converting 3-(3-indolyl)propionic acid free acid into a calcium salt;
(b) precipitating and washing the calcium salt; and
(c) reconverting the calcium salt into the free acid.

3. The method of claim 2, wherein the substantially pure 3-(3-indolyl)propionic acid free acid has a purity of 97% or greater.

4. The method of claim 3, wherein the substantially pure 3-(3-indolyl)propionic acid free acid has a purity of 99% or greater.

## Patentansprüche

1. 3-(3-Indolyl)propionsäure-Calciumsalz oder ein Hydrat davon.

2. Verfahren zum Herstellen von im Wesentlichen reiner 3-(3-Indolyl)propionsäure in Form der freien Säure, umfassend die Schritte:
(a) das Umwandeln von 3-(3-Indolyl)propionsäure in Form der freien Säure in ein Calciumsalz;
(b) das Ausfällen und Waschen des Calciumsalzes; und
(c) das Rückumwandeln des Calciumsalzes in die freie Säure.

3. Verfahren nach Anspruch 2, wobei die im Wesentlichen reine 3-(3-Indolyl)propionsäure in Form der freien Säure eine Reinheit von 97 % oder mehr aufweist.

4. Verfahren nach Anspruch 3, wobei die im Wesentlichen reine 3-(3-Indolyl)propionsäure in Form der freien Säure eine Reinheit von 99 % oder mehr aufweist.

## Revendications

1. Sel de calcium de l'acide 3-(3-indolyl)-propionique, ou hydrate dudit sel.

2. Procédé de préparation de la forme acide libre de l'acide 3-(3-indolyl)-propionique sous forme sensiblement pure, comprenant les étapes de :
(a) conversion de la forme acide libre de l'acide 3-(3-indolyl)-propionique en un sel de calcium ;
(b) précipitation et rinçage du sel de calcium ; et
(c) reconversion du sel de calcium en l'acide libre.

3. Procédé selon la revendication 2, dans lequel la forme acide libre de l'acide 3-(3-indolyl)-propionique sous forme sensiblement pure a une pureté de 97 % ou plus.

4. Procédé selon la revendication 3, dans lequel la forme acide libre de l'acide 3-(3-indolyl)-propionique sous forme sensiblement pure a une pureté de 99 % ou plus.
